(19) Europäisches Patentamt
European Patent Office
Office européen des brevets

(11) Publication number: 0 477 700 A1

(12) **EUROPEAN PATENT APPLICATION**

(21) Application number: 91115578.6

(51) Int. Cl.$^5$: C07D 473/34

(22) Date of filing: 13.09.91

(30) Priority: 17.09.90 JP 246791/90

(43) Date of publication of application:
01.04.92 Bulletin 92/14

(84) Designated Contracting States:
AT BE CH DE DK ES FR GB GR IT LI LU NL SE

(71) Applicant: TOYO JOZO CO., LTD.
632-1, Mifuku Ohito-cho
Tagata-gun Shizuoka 410-23(JP)

(72) Inventor: Shuto, Satoshi
690-10, Makinokou, Shuzenji-cho
Tagata-gun, Shizuoka 410-24(JP)
Inventor: Obara, Takumi
59-1, Odoi, Kannami-cho
Tagata-gun, Shizuoka 419-01(JP)
Inventor: Fujiwara, Tatsuro
310, Nirayama, Nirayama-cho
Tagata-gun, Shizuoka 410-21(JP)
Inventor: Toriya, Minoru
854-1, Mifuku, Ohito-cho
Tagata-gun, Shizuoka 410-23(JP)
Inventor: De Clercq, Erik
Parklaan 9
B-3042 Bierbeek-Lovenjoel(BE)

(74) Representative: Wächtershäuser, Günter, Dr.
Tal 29
W-8000 München 2(DE)

(54) **6'-C-alkyl- or alkynyl-neplanocin A, and its preparation process and use.**

(57) 6'-C-Alkyl- or alkynyl-neplanocin A compounds and their salts are described. They are represented by the following formula (I):

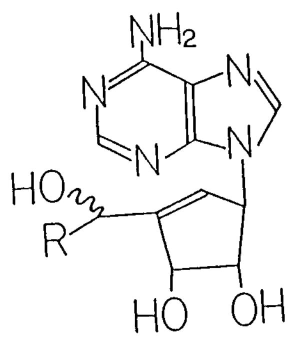

(I)

wherein R represents a lower alkyl group or a lower alkynyl group. Their preparation processes and antiviral agents containing them or their salts are also described.

EP 0 477 700 A1

## BACKGROUND OF THE INVENTION

1) Field of the Invention

This invention relates to novel derivatives of neplanocin A, and more specifically to neplanocin A derivatives useful as drugs, especially as antiviral agents.

2) Description of the Related Art

Neplanocin A is an antibiotic having antitumor activity and growth inhibitory activity for plant pathogenic fungi, i.e., filamentous fungi. It is produced by a microorganism, *Ampullariella sp.* A11079, and is represented by the following formula (II):

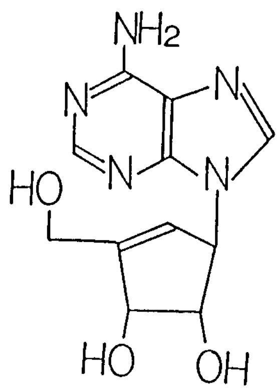

(II)

(Japanese Patent Application Laid-Open No. 154792/1979). It has recently found that this compound also has antiviral activity (ANTIMICROBIAL AGENTS AND CHEMOTHERAPY, 84-89. July 1985). Its use as an antiviral agent is therefore under investigation.

Neplanocin A is, however, accompanied by the drawback that it is susceptible to the action of adenosine deaminase present abundant in organisms, resulting in prompt inactivation. This has remained as a problem for its utilization as a drug.

In addition, the high citotoxity level of neplanocin A has also been an unignorable problem upon its use as a drug.

## SUMMARY OF THE INVENTION

The present inventors have carried out an extensive investigation with a view toward overcoming the above-described problems of neplanocin A and obtaining an excellent antiviral agent. As a result, it has been found that alkylation or alkynylation at the 6-carbon of neplanocin A can improve its resistance to adenosine deaminase without reducing its antiviral activity and neplanocin A derivatives so obtained have low cytotoxicity, leading to the completion of the present invention.

An object of the present invention is therefore to provide a 6'-C-alkyl- or alkynyl-neplanocin A represented by the following formula (I):

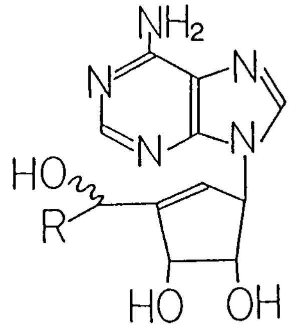

(I)

wherein R represents a lower alkyl group or a lower alkynyl group, or a salt thereof.

Another object of the present invention is to provide a process for the preparation of the 6'-C-alkyl- or alkynyl-neplanocin A (I).

A further object of the present invention is to provide an antiviral agent which contains the 6'-C-alkyl- or alkynyl-neplanocin A (I) as an active ingredient.

In one aspect of the present invention, there is thus provided the 6'-C-alkyl- or alkynyl-neplanocin A (I) or a salt thereof.

In another aspect of the present invention, there is also provided a process for the preparation of the 6'-C-alkyl- or alkynyl-neplanocin A (I) or a salt thereof, which comprises removing the protecting groups of a protected 6'-C-alkyl- or alkynyl-neplanocin A represented by the following formula (V):

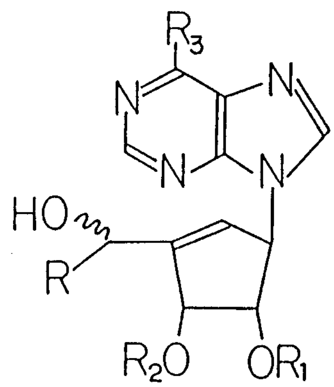

(V)

wherein R has the same meaning as defined above, $R_1$ and $R_2$ individually or in combination mean a protecting group for a hydroxyl group, and $R_3$ denotes a protected amino group.

In a further aspect of the present invention, there is also provided a process for the preparation of the 6'-C-alkyl- or alkynyl-neplanocin A (I) or a salt thereof, which comprises reacting a carbon-nucleophile with a 6'-formyl derivative represented by the following formula (IV):

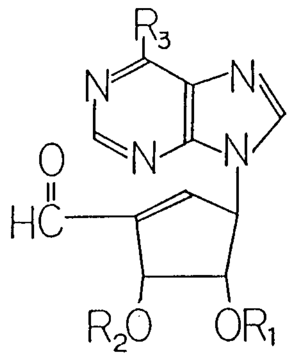

(IV)

wherein $R_1$, $R_2$ and $R_3$ have the same meanings as defined above, whereby a C-substituent group is introduced to obtain the protected 6'-C-alkyl- or alkynyl-neplanocin A (V):

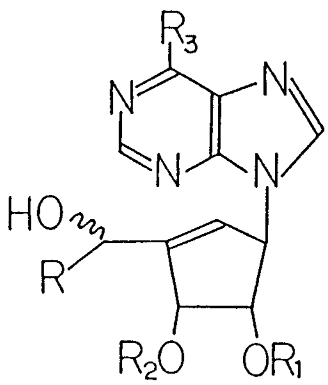

(V)

wherein R, $R_1$, $R_2$ and $R_3$ have the same meanings as defined above, and removing the protecting groups.

In a still further aspect of the present invention, there is also provided a process for the preparation of the 6'-C-alkyl- or alkynyl-neplanocin A (I) or a salt thereof, which comprises oxidizing a protected neplanocin A represented by the following formula (III):

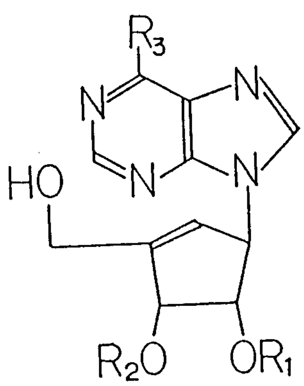

(III)

wherein $R_1$, $R_2$ and $R_3$ have the same meanings as defined above, thereby obtaining the 6'-formyl derivative (IV), reacting the 6'-formyl derivative with a carbon-nucleophile, whereby a C-substituent group is

introduced to obtain the protected 6'-C-alkyl- or alkynyl-neplanocin A (V), and removing the protecting groups.

In a still further aspect of the present invention, there is also provided an antiviral agent comprising as an active ingredient the 6'-C-alkyl- or alkynyl-neplanocin A (I) or a salt thereof.

In a still further aspect of the present invention, there is also provided a compound represented by the following formula (VI):

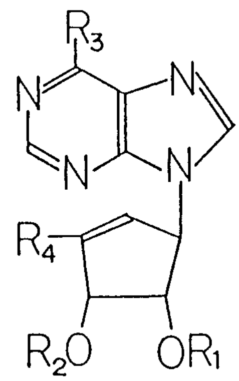

(VI)

wherein $R_1$, $R_2$ and $R_3$ have the same meanings and $R_4$ represents:

group 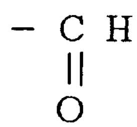 or group 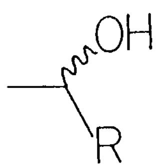

wherein R has the same meaning as defined above.

The 6'-C-alkyl- or alkynyl-neplanocin A (I) of the present invention can be advantageously used as an antiviral agent.

## DETAILED DESCRIPTION OF THE INVENTION

The lower alkyl group in the 6'-C-alkyl- or alkynyl-neplanocin A of the present invention can be a normal or branched alkyl group having about 1-4 carbon atoms. Specific examples include methyl, ethyl, n-propyl, i-propyl, n-butyl, i-butyl, tert-butyl, and the like.

On the other hand, the lower alkynyl group can be a normal or branched alkynyl group having about 2-4 carbon atoms. Specific examples include ethynyl, 1-(or 2-)propynyl, 1-(or 2- or 3-)butynyl, etc.

The 6'-C-alkyl- or alkynyl-neplanocin A of the present invention can be prepared, for example, in accordance with the following reaction scheme, namely, by protecting the 6-amino group and 2'- and 3'-hydroxyl groups of neplanocin A (II) to obtain an amino-protected derivative (III), oxidizing the amino-protected derivative into a 6'-formyl derivative (IV), reacting the formyl derivative (IV) with a carbon-nucleophile to introduce a 6'-C-substituent group and hence to obtain a compound (V), and then removing the amino-protecting group and the hydroxyl-protecting groups.

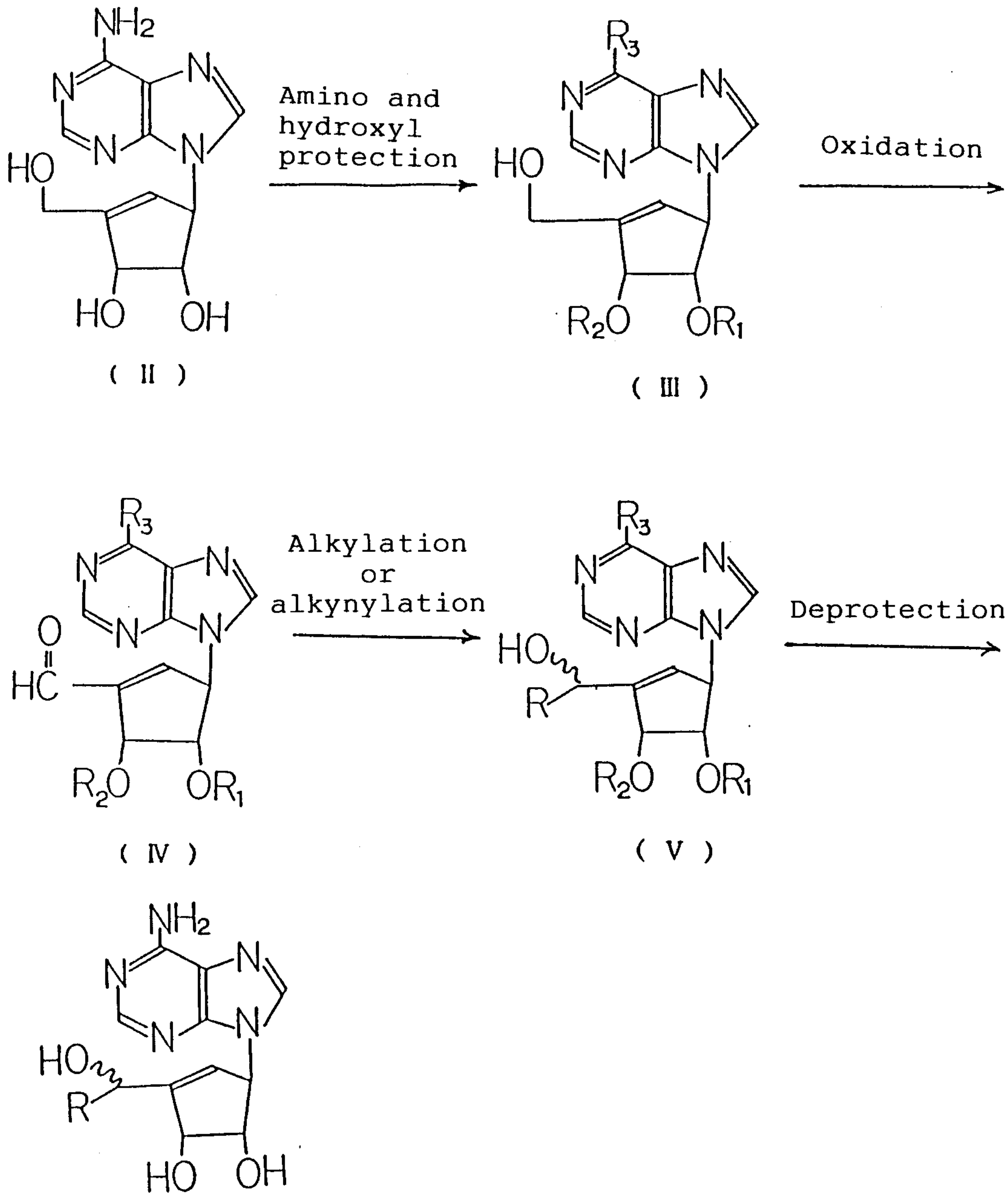


( II ) ( III )

( IV ) ( V )

( I )

wherein $R_1$ and $R_2$ individually or in combination mean the protecting group for the hydroxyl group, and $R_3$ denotes the protected amino group, and R has the same meaning as defined above.

In the above reaction scheme, no particular limitation is imposed on the protection of the amino group and hydroxyl groups of neplanocin A (I). Their protection can be effected usually by a method employed in sugar or nucleic acid chemistry.

Particularly preferred amino-protecting groups are those remaining stable under oxidizing and alkylating reaction conditions and permitting easy introduction and deprotection, including acyl-protecting groups such as benzoyl and phthaloyl groups and urethane-type protecting groups such as benzyloxycarbonyl and t-butoxycarbonyl groups.

Preferred hydroxyl-protecting groups include protecting groups capable of simultaneously protecting the 2'- and 3'-hydroxyl groups, such as isopropylidene, benzylidene and ethoxymethylene groups; and silyl protecting groups such as t-butyldimethylsilyl and di-isopropylmethylsilyl groups, and ether-type protecting groups such as benzyl and methoxymethyl groups. The oxidation of the amino-protected derivative (III) can be done by known procedures for oxidizing a hydoxyl group to a formyl group which have been used in organic chemistry. Particularly preferred method is conducted in an inert solvent, using an oxidizing agent

such as an oxidizing agent of the chromic acid or manganic acid type. Namely, the oxidation reaction is conducted at a temperature ranging from room temperature to the reflux temperature in an inert solvent such as methylene chloride, chloroform or dichloroethane, using an excess amount of an oxidizing agent such as $MnO_2$ or $BaMnO_4$. The 6'-formyl derivative (IV) obtained by the above-described oxidation reaction is then reacted with a carbon-nucleophile to introduce a 6'-C-substituent group, whereby the derivative (IV) is converted to the compound (V). Subsequent deprotection provides the 6'-C-alkyl- or alkynyl-neplanocin A of the present invention.

The introduction of the C-substituent group can be effected by reacting - as the carbon-nucleophile - an alkylating reagent, for example, a Grignard reagent such as methylmagnesium bromide or ethylmagnesium bromide, an alkyllithium such as methyllithium or ethyllithium, a trialkylaluminum such as trimethylaluminum or triethylaluminum, an organotitanium reagent such as trimethoxymethyltitanium and dichlorodimethyltitanium, or an organozinc reagent such as dimethylzinc or diethylzinc; or an alkynylating agent such as a Grignard reagent such as ethynylmagnesium bromide, an alkynyllithium such as ethynyllithium, an organoaluminum such as triethynylaluminum, or an organozinc reagent such as diethynylzinc, to the 6'-formyl derivative (IV) in an inert solvent such as methylene chloride, an ether solvent such as THF or a hydrocarbon solvent such as hexane.

The end hydrogen atom of the alkynylating reagent may be protected, for example, by a silyl group such as trimethylsilyl group.

Further, the removal of the amino-protecting group and hydroxyl-protecting groups from the compound (V) can be conducted usually by a method employed in sugar or nucleic acid chemistry.

To obtain the thus-prepared 6'-C-alkyl- or alkynyl-neplanocin A (I) from the reaction mixture, conventional separating and purification methods can be used. The compound (I) can be separated and purified by column chromatography, namely, by distilling off the solvent employed in the reaction, dissolving the residue in a solvent such as methanol, adsorbing the compound on an adsorbent such as silica gel and then eluting it with an elution solvent such as a chloroform-methanol solvent system.

The 6'-C-alkyl- or alkynylneplanocin A (I) can be converted to pharmaceutically-acceptable, non-toxic salts as needed, by a method known *per se* in the art.

Examples of such salts include inorganic acid salts such as the hydrochloride, sulfate and phosphate; and organic acid salts such as the acetate, propionate, tartrate, citrate, glycolate, gluconate, succinate, malate, glutamate, aspartate and methanesulfonate.

To use the invention compound (I) or its salt as an antiviral agent, it is only necessary to administer an effective amount of the compound (I) or its salt as is or after formulating the same together with a known carrier into a dosage form.

Usable administration methods include oral administration, rectal administration, parenteral administration (for example, intravascular, intramuscular, subcutaneous or intraperitoneal administration), and transocular administration. The above-mentioned formulation can be conducted depending on the administration method. Dosage forms include, for example, tablets, pills, powder, granules, capsules, injection, suppositories and eye drop. For their formulation, various carriers corresponding to the individual dosage forms can be used. For example, oral preparations such as tablets, granules and capsules can use excipients such as starch, lactose, sucrose, mannitol, carboxymethylcellulose, corn starch and inorganic salts; binders such as starch, dextrin, gum arabic, gelatin, hydroxypropylstarch, methylcellulose, sodium carboxymethylcellulose, hydroxypropylcellulose, crystalline cellulose, ethylcellulose, polyvinyl pyrrolidone and macrogol; disintegrators such as starch, hydroxypropylstarch, carboxymethylcellulose, sodium carboxymethylcellulose and hydroxypropylcellulose; surfactants such as sodium lauryl sulfate, soybean lecithin, sucrose fatty acid ester and Polysolvate 80; lubricants such as talc, wax, a hydrogenated vegetable oil, sucrose fatty acid ester, magnesium stearate and calcium stearate; fluidity promoters; corrigents; colorants; and perfumes.

The invention compound (I) or its salt can also be used in the form of a suspension, emulsion, syrup or elixir.

A parenteral preparation can generally use, as a diluent, a distilled water for injection, physiological saline, an aqueous glucose solution, a vegetable oil for injection, propylene glycol, polyethylene glycol or the like. Further, antiseptic, preservative and/or stabilizer can also be added as needed. From the safety standpoint, it is also possible to fill and lyophilize the parenteral preparation in a vial or the like and, immediately before use, to reconstitute it with a diluent. In addition, isotonicity, stabilizer, preservative, soothing agent and the like can also be incorporated suitably as needed.

The dose of the invention compound (I) or its salt varies depending on the administration route, the age, body weight and conditions of the patient, etc. In general, the daily dose may be about 5 mg to 1 g, preferably, about 25-300 mg per adult in term of the compound (I). It is administered in 1-3 portions.

Some 6'-C-alkyl- or alkynyl-neplanocin A (I) which had been obtained as described above were tested

for their pharmacological effects. The results will be described next.

(1) Antiviral activity and cytotoxicity against parainfulenza virus:

Using parainfulenza virus as a virus and Vero cells (product of Flow General Inc., marketed by Dainippon Pharmaceutical Co., Ltd., available from National Institute of Health) as cells, the minimum virus growth inhibitory concentration (MIC) and minimum cytotoxicity concentration (MCC) of each test compound were investigated by the methods described in ANTIMICROBIAL AGENTS AND CHEMOTHERAPY, **28**(1), 84-89 (1985). The results are shown in Table 1.

Table 1

| Compound | MIC (μg/mℓ) | MCC (μg/mℓ) |
|---|---|---|
| 6'-C-Methylneplanocin A | 0.1 | ≧400 |
| Neplanocin A | 0.1 | ≧40 |

(2) Vesicular Stomatitis Virus (VSV) growth inhibitory activity:

By partly modifying the testing method disclosed in ANTIMICROBIAL AGENTS AND CHEMOTHERAPY, **24**(3), 353-361 (1983), antiviral activity was analyzed in the following manner. 200 μl of Eagle's minimum essential medium (with 10% fetal calf serum), in which Vero cells were suspended at the concentration of 3 x $10^5$ cells/mℓ, were cultured in a 98-well flat bottom plate. Subsequent to the dense monolayer culture, the medium was removed and 90 μℓ of Eagle's minimum essential medium (with 3% fetal calf serum) containing a test compound were added. The tissue culture infections dosis 50% ($TCID_{50}$) had been determined in advance without addition of any drug. The above-prepared culture plate was inoculated with 10 μℓ of a virus solution whose concentration was 100 times higher than $TCID_{50}$ determined in advance. The cells were cultured for 36 hours at 37°C under 5% $CO_2$. The cytopathic effect (CPE) was microscopically observed, whereby the minimum virus growth inhibitory concentration (MIC) was determined. The results are shown in Table 2.

Table 2

| Compound | MIC (μg/mℓ) |
|---|---|
| 6'-C-Methylneplanocin A | 0.1 |
| 6'-C-Ethynylneplanocin A | 0.1 |
| Neplanocin A | 3.9 |

(3) Adenosine deaminase resistance:

Each test compound was dissolved at 5 mM concentration in 0.05 M Tris-HCl buffer (pH 7.2) to provide a test solution. On the other hand, adenosine deaminase (calf intestine, 400 U/mℓ glycerin, Boehringer 102091) was diluted tenfold in 0.05 M Tris-HCl buffer (pH 7.2) to provide an adenosine deaminase (AD) solution.

The AD solution (10 μℓ) was added to 0.5 mℓ of the test solution, followed by mixing. The resultant mixture was left over in a water bath which was controlled at 25°C. Upon elapsed times of 0 and 30 minutes, 4-μℓ portions were sampled respectively. The amount of the test compound still remaining in each sample solution was measured by high performance liquid chromatography (HPLC) under the following conditions. The results are summarized in Table 3.

(HPLC conditions)

Column: Superspher RP-18-4

Elution solvent: 15% methanol
Elution velocity: 1.0 mℓ/min
Temperature: 50 ° C

(Test results)

Table 3

| Compound | Percent remainder | |
|---|---|---|
| | 0 minute | 30 minutes |
| Neplanocin A (Control) | 100 | 0 |
| 6'-C-Methylneplanocin A | 100 | 92 |
| 6'-C-Ethylneplanocin A | 100 | 94 |
| 6'-C-Ethynylneplanocin A | 100 | 96 |

Incidentally, no case of death was observed by 200 mg/kg oral administration of the 6'-C-alkyl- or alkynylneplanocin A (I) of the present invention.

As is apparent from the results of the above-described tests on pharmacological activities, the 6'-C-alkyl- or alkynyl-neplanocin A (I) of the present invention exhibits excellent antiviral activity and, moreover, its cytotoxicity is lower compared to neplanocin A. Further, as is understood clearly from the fact the invention compound was inactivated as little as several % under the conditions where neplanocin A was inactivated 100%, the 6'-C-alkyl- or alkynyl-neplanocin A (I) of the present invention shows strong resistance to adenosine deaminase and also retain high stability in organisms.

The 6'-C-alkyl- or alkynyl-neplanocin A (I) of the present invention can therefore be advantageously used as an antiviral agent.

The present invention will next be described in further detail by the following examples.

Example 1

Preparation of $N^6$-benzoylneplanocin A

Neplanocin A (3.16 g, 12 mmol) was suspended in 60 mℓ of pyridine, followed by the addition of 7.6 mℓ (60 mmol) of TMSCl (trimethylsilyl chloride). After the resultant mixture was stirred at room temperature for 15 minutes, 7.0 mℓ (60 mmol) of benzoyl chloride were added, followed by further stirring at room temperature for 2 hours. The reaction mixture was ice-cooled, to which 12 mℓ of water were added. Subsequent to stirring for 5 minutes, 24 mℓ of concentrated aqueous ammonia were added and the resulting mixture was stirred at room temperature for 45 minutes. The solvent was distilled off under reduced pressure, the residue was taken up in 60 mℓ of ethyl acetate and 180 mℓ of water. The water layer was concentrated under reduced pressure. An insoluble matter so formed was collected by suction filtration, whereby 2.75 g of $N^6$-benzoylneplanocin A were obtained.

On the other hand, the filtrate was purified by chromatography on a silica gel column (solvent system: $CHCl_3$:MeOH = 20:1 → 15:1), whereby 0.87 g of $N^6$-benzoylneplanocin A was additionally obtained. Total yield: 3.62 g (83%).

$^1$H-NMR (DMSO-$d_6$, 400 MHz):

δ: 8.73,8.42(each s, each 1H, H-2 and H-8), 8.06-7.54(m,5H,Bz), 5.76(d,1H,H-5',J = 1.5Hz), 5.51-(m,1H,H-1'), 5.22,5.01(each d, each 1H, 2'- and 3'-OH). 4.95(t,1H,b'-OH), 4.46(m,1H, H-3'), 4.38-(m,1H,H-2'), 4.15(m,2H,H-6').

FAB-MS(m/e): 368 ($MH^+$)

Example 2

Preparation of $N^6$-benzoyl-2',3'-O-isopropylideneneplanocin A

$N^6$-Benzoylneplanocin A (1.47 g, 4 mmol) was suspended in 100 mℓ of acetone, followed by the

addition of 1.0 mℓ of 70% $HClO_4$. The resultant mixture was stirred at room temperature for 3 hours. It was neutralized with 0.75N $NaHCO_3$. An insoluble matter so formed was collected by suction filtration and the filtrate was evaporated to dryness under reduced pressure. The residue was taken up in $CHCl_3$ and saline. The water layer as extracted further with $CHCl_3$. The $CHCl_3$ layers were combined, dried over anhydrous $MgSO_4$ and then filtered. The filtrate was evaporated to dryness under reduced pressure. The residue was crystallized from ether, whereby 1.25 g of $N^6$-benzoyl-2',3'-O-isopropylideneneplanocin A were obtained (yield: 77%).

$^1$H-NMR ($CDCl_3$, 100 MHz):

δ: 8.76,8.31 (each s, each 1H, H-2 and H-8), 8.10-7.40(m,5H,Bz), 5.77(bs,1H,H-5'), 5.61(bs,1H,H-1'), 5.88(d,1H,H-3',J=5.6Hz), 5.11(broad,1H,6'-OH), 4.79(d,1H,H-2'), 4.18(bs,2H,H-6'), 1.42,1.30(each s, each 3H, isopropylidene$CH_3$).

Example 3

Preparation of $N^6$-benzoyl-2',3'-O-isopropylidene-6'-C-methylneplanocin A

1,2-Dichloroethane (300 mℓ) was added to the mixture of 1.25 g (3 mmol) of $N^6$-benzoyl-2'.3'-O-isopropylideneneplanocin A and 18 g of $BaMnO_4$. The resultant mixture was refluxed for 3 hours. The reaction mixture was filtered by means of suction, and the filtrate was evaporated to dryness under reduced pressure so that 0.89 g of a residue was obtained.

The residue was dissolved in 50 mℓ of benzene. After being purged with argon, the resultant solution was evaporated to dryness under reduced pressure. The residue was dissolved in 30 mℓ of $CH_2Cl_2$. The reaction system was cooled to -70°C or lower and 6 mℓ of a $Me_3Al$ solution (1.38 N, hexane) were poured. The resultant mixture was stirred for 2 hours at the same temperature. 1N $NH_4Cl$ (20 mℓ) was added and the internal temperature was raised to room temperature. Subsequent to the addition of 8 mℓ of 4N HCl, the resulting mixture was allowed to separate into layers. After the organic layer was washed with saline, anhydrous $Na_2SO_4$ was added to dry the organic layer. The $Na_2SO_4$ was then filtered off. The solvent was distilled off. The residue was concentrated and then purified by chromatography on a silica gel flash column (2.5 x 15 cm, $CHCl_3$ → $CHCl_3$:MeOH = 100:1 → 80:1), whereby 0.50 g of $N^6$-benzoyl-2'.3'-O-isopropylidene-6'-C-methylneplanocin A was obtained (yield: 38%).

FAB-MS (m/e): 422 ($MH^+$)

Example 4

Preparation of 2',3'-O-isopropylidene-6'-C-methylneplanocin A

$N^6$-Benzoyl-2',3'-O-isopropylidene-6'-C-methylneplanocin A (152 mg, 0.35 mmol) was dissolved in 3 mℓof dioxane, followed by the addition of 3 mℓ of concentrated aqueous ammonia. The resultant mixture was stirred overnight at room temperature. The solvent was distilled off under reduced pressure and the residue was dissolved in a small amount of methanol. Silica gel (1.0 g) was added, followed by evaporation to dryness under reduced pressure. The resulting powder was applied to a silica gel flash column (1.5 cm in diameter x 12 cm in height, $CHCl_3$ → $CHCl_3$:MeOH = 50:1 → 30:1 → 10:1), whereby the powder was Purified and 96 mg of 2',3'-O-isopropylidene-6'-C-methylneplanocin A were obtained (yield: 84%).

FAB-MS (m/e): 318 ($MH^+$).

Example 5

Preparation of 6'-C-methylneplanocin A

2',3'-O-Isopropylidene-6'-C-methylneplanocin A (92 mg, 0.29 mmol) was dissolved in 3 mℓ of 90% formic acid, followed by stirring at room temperature for 3 hours. The solvent was distilled off under reduced pressure. A small amount of MeOH was added to the residue to dissolve the latter. The resultant solution was added with 600 mg of silica gel and then evaporated to dryness under reduced pressure. The resultant powder was applied to a silica gel flash column (1.5 cm in diameter x 8 cm in height). The column was developed and eluted in the order of $CHCl_3$ → $CHCl_3$:MeOH = 20:1 → 10:1 → 4:1, whereby 68 mg of 6'-C-methylneplanocin A were obtained (yield: 84%).

FAB-MS (m/e): 278 ($MH^+$).

$^1$H-NMR (DMSO-$d_6$ added with $D_2O$; 400 MHz):

δ: 8.12,8.07,8.04(each s,H-2 and H-8), 5.70,5.68(each m,H-5'), 5.36-5.33(m,H-1'), 4.51,4.46(each d,H-3'), 4.35-4.24(m, H-2' and H-6'), 1.27,1.26(each d,6'-$CH_3$).

Example 6

Separation of 6'-C-methylneplanocin A diastereomers

Dissolved in 2 mℓ of water were 61 mg of the 6'-C-methylneplanocin A, i.e., 6'-position diastereomer mixture obtained in Example 5. The resultant solution was fractionated by preparative HPLC (column: Merck, "Lichrosorb RP-18-5", 2.5 x 50 cm, solvent: 7% aqueous methanol solution). Each eluate fraction was analyzed by HPLC (column: E. Merck AG, "Supersher RP18-4", 0.4 x 15 cm, solvent: 12% aqueous methanol solution, temperature: 50 ° C, flow rate: 1 mℓ/min, detection: UV 260 nm). The fraction having a peak at 3.7 minutes of the elution time and that having a peak at 4.2 minutes of the elution time were separately concentrated under reduced pressure. Diastereomer A (40 mg) was obtained from the fraction eluted earlier, while diastereomer B (12 mg) was obtained from the fraction eluted subsequently.

(Physical properties of diastereomer A)

Melting point: 211 ° C.
$^1$H-NMR (DMSO-$d_6$ added with $D_2O$; 400 MHz):
δ: 8.11,8.04(each s, 1H, H-2 and H-8), 5.68(m,1H,H-5'), 5.33(m,1H,H-1'), 4.49(d,1H,H-3'), 4.33(m,1H,H-6'), 4.22(dd,1H,H-2'), 1.25(d,3H,6'-$CH_3$).
FAB-MS (m/e): 278 ($MH^+$)

(Physical properties of diastereomer B)

Melting point: 231 ° C.
$^1$H-NMR (DMSO-$d_6$ added with $D_2O$; 400 MHz):
δ: 8.10,8.09(each s, 1H, H-2 and H-8), 5.70(m,1H,H-5'), 5.34(m,1H,H-1'), 4.44(d,1H,H-3'), 4.27-4.23-(m,2H,H-2',H-6'), 1.26(d,3H,6'-$CH_3$).
FAB-MS (m/e): 278 ($MH^+$)

Example 7

Preparation of 6'-C-ethylneplanocin A

(1) $N^6$-benzoyl-2',3'-O-isopropylidene-6'-C-ethylneplanocin A was obtained by using $Et_3Al$ instead of $Me_3Al$ in Example 3.
Yield: 210 mg (76%).
FAB-MS (m/e): 436 ($MH^+$).
(2) $N^6$-Benzoyl-2',3'-O-isopropylidene-6'-C-ethylneplanocin A (35 mg) was dissolved in 2 mℓ of dioxane, to which 2 mℓ of concentrated aqueous ammonia were added. The resultant mixture was stirred at room temperature for 18 hours. The reaction mixture was concentrated under reduced pressure and the residue was dissolved in 2 mℓ of 90% aqueous formic acid solution. The resultant solution was stirred at room temperature for 2 hours and then evaporated to dryness under reduced pressure. The procedure in which water was added to the residue followed by evaporation to dryness was repeated twice. Concentrated aqueous ammonia (2 mℓ) was added to the residue, followed by stirring for 5 minutes. The solvent was distilled off under reduced pressure and the residue was dissolved in a small amount of methanol. The resultant solution was added with 300 mg of silica gel and then evaporated to dryness under reduced pressure. The resultant powder was applied to a silica gel flash column (1.5 cm in diameter x 8 cm in height). The column was developed and eluted in the order of chloroform → chloroform: methanol = 20:1 → 10:1 → 4:1, whereby 16 mg of 6'-C-ethylneplanocin A were obtained (yield: 68%).
$^1$H-NMR ($CDCl_3$, 400 MHz):
δ: 8.18,8.10,8.06(each s,H-2,H-8), 5.91-5.89(m,H-5'), 5.52-5.48(m,H-1'), 4.70-4.65(m,H-3'), 4.39-4.21-(m,H-2',H-6'), 1.90-1.61(m,2H,-$\underline{CH_2}CH_3$),1.05-1.00(m,3H,-$CH_2\underline{CH_3}$).
FAB-MS (m/e): 292 ($MH^+$).

Example 8

Preparation of 6'-C-ethynylneplanocin A (1) $N^6$-Benzoyl-6'-C-ethynyl-2',3'-O-isopropylideneneplanocin A

1,2-Dichloroethane (300 mℓ) was added to the mixture of 1.25 g of $N^6$-benzoyl-2',3'-O-isopropylideneneplanocin A and 18 g of $BaMnO_4$, and the resultant mixture was refluxed for 3 hours. The reaction mixture was filtered by means of suction. The filtrate was evaporated to dryness under reduced pressure, whereby 0.89 g of a residue was obtained.

Ninety milligrams of the residue were dissolved in 5 mℓ of tetrahydrofuran. After the reaction system was purged with argon gas, 0.75 mℓ of an ethynyl magnesium bromide solution (0.5 M, tetrahydrofuran) was poured while the reaction system was cooled at -15 °C. At the same temperature, the resultant mixture was stirred for 2 hours. Acetic acid (100 μℓ) was added to the reaction mixture and the mixture so prepared was evaporated to dryness under reduced pressure. Chloroform and saline were added to the residue, and the resulting mixture was allowed to separate into layers. The organic layer was washed with saturated saline, dried over anhydrous sodium sulfate, and then evaporated to dryness under reduced pressure. The residue was purified through a silica gel flash column (chloroform: methanol = 120:1 → 80:1), whereby 35 mg of $N^6$-benzoyl-6'-C-ethynyl-2',3'-O-isopropylideneneplanocin A were obtained (yield 41%).
FAB-MS (m/e): 432 ($MH^+$)

(2) 6'-Ethynylneplanocin A

$N^6$-Benzoyl-6'-C-ethynyl-2',3'-O-isopropylideneneplanocin A (35 mg) was dissolved in 2 mℓ of dioxane, followed by the addition of 2 mℓ of concentrated aqueous ammonia. The resultant mixture was stirred at room temperature for 18 hours. The reaction mixture was evaporated to dryness under reduced pressure and the residue was dissolved in 2 mℓ of 90% aqueous formic acid solution. The solution was stirred at room temperature for 2 hours and then evaporated to dryness under reduced pressure. The procedure in which water was add to the residue followed by evaporation to dryness was repeated twice. Concentrated aqueous ammonia (2 mℓ) was added to the residue, followed by stirring for 5 minutes. The solvent was distilled off under reduced pressure and the residue was dissolved in a small amount of methanol. The resultant solution was added with 300 mg of silica gel and then evaporated to dryness under reduced pressure. The resultant powder was applied to a silica gel flash column (1.5 cm in diameter x 8 cm in height). The column was developed and eluted in the order of chloroform → chloroform: methanol = 20:1 → 10:1 → 4:1, whereby 9 mg of 6'-C-ethynylneplanocin A were obtained (yield: 39%).
FAB-MS (m/e): 288 ($MH^+$).
$^1$H-NMR ($CD_3OD$, 400 MHz):
δ: 8.20,8.19,8.10,8.08(each s,H-2,H-8), 6.21,6,.07(each m,H-5'), 5.55-5.50(m,H-1'), 5.13,5.05(each m,H-3'), 4.77,4.58(each m,H-6'), 4.43-4.39(m,H-2'), 2.99(m,ethynyl group).

**Claims**

**1.** A 6'-C-alkyl- or alkynyl-neplanocin A represented by the following formula (I):

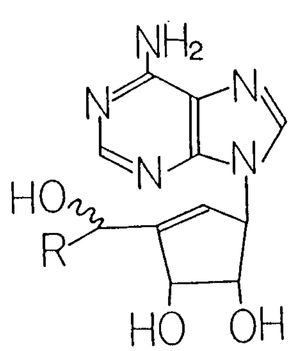

(I)

wherein R represents a lower alkyl group or a lower alkynyl group, or a salt thereof.

**2.** A process for the preparation of a 6'-C-alkyl- or alkynyl-neplanocin A represented by the following

formula (I):

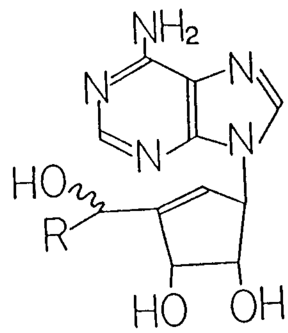

(I)

wherein R represents a lower alkyl group or a lower alkynyl group, or a salt thereof, which comprises:
removing the protecting groups of a protected 6'-C-alkyl-or alkynyl-neplanocin A represented by the following formula (V):

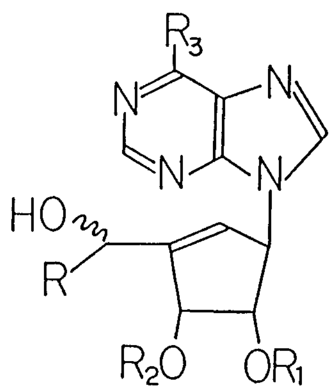

(V)

wherein R has the same meaning as defined above, $R_1$ and $R_2$ individually or in combination mean a protecting group for a hydroxyl group, and $R_3$ denotes a protected amino group.

**3.** A process for the preparation of a 6'-C-alkyl- or alkynyl-neplanocin A represented by the following formula (I):

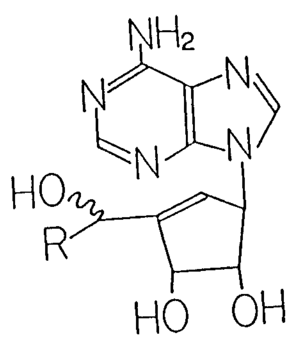

(I)

wherein R represents a lower alkyl group or a lower alkynyl group, or a salt thereof, which comprises:

reacting a carbon-nucleophile with a 6'-formyl derivative represented by the following formula (IV):

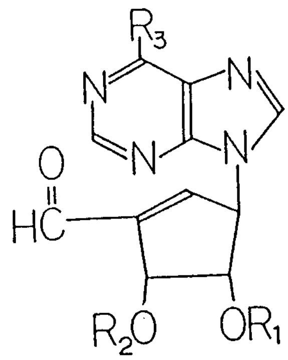

(IV)

wherein $R_1$ and $R_2$ individually or in combination mean a protecting group for a hydroxyl group and $R_3$ denotes a protected amino group, whereby a C-substituent group is introduced to obtain a protected 6'-C-alkyl- or alkynyl-neplanocin A represented by the following formula (V):

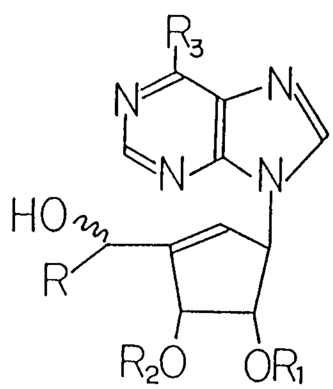

(V)

wherein R, $R_1$, $R_2$ and $R_3$ have the same meanings as defined above; and removing the protecting groups.

**4.** A process for the preparation of a 6'-C-alkyl- or alkynyl-neplanocin A represented by the following formula (I):

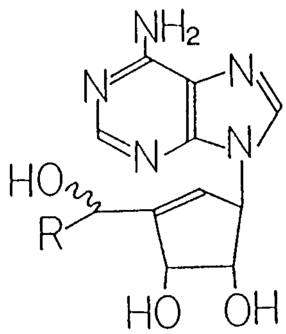

(I)

wherein R represents a lower alkyl group or a lower alkynyl group, or a salt thereof, which comprises:

oxidizing a protected neplanocin A represented by the following formula (III):

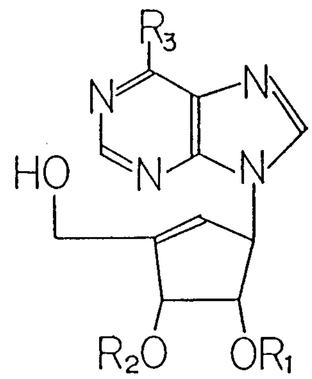

(III)

wherein $R_1$ and $R_2$ individually or in combination mean a protecting group for a hydroxyl group and $R_3$ denotes a protected amino group, thereby obtaining a 6'-formyl derivative represented by the following formula (IV):

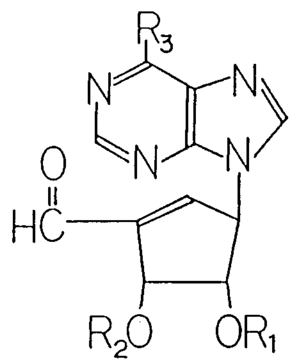

(IV)

wherein $R_1$, $R_2$ and $R_3$ have the same meanings as defined above;

reacting the 6'-formyl derivative with a carbon-nucleophile, whereby a C-substituent group is introduced to obtain a protected 6'-C-alkyl- or alkynylneplanocin A represented by the following formula (V):

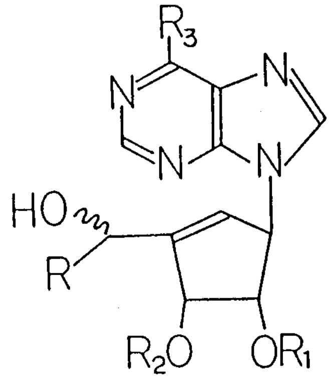

(V)

wherein R, $R_1$, $R_2$ and $R_3$ have the same meanings as defined above; and removing the protecting groups.

**5.** An antiviral agent comprising as an active ingredient a 6'-C-alkyl- or alkynyl-neplanocin A represented by the following formula (I):

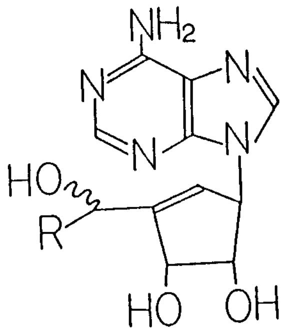

(I)

wherein R represents a lower alkyl group or a lower alkynyl group, or a salt thereof.

**6.** A compound represented by the following formula (VI):

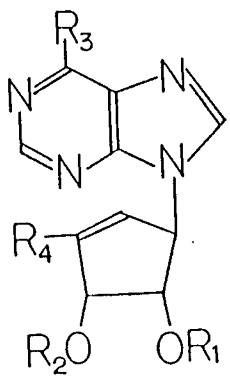

(VI)

wherein $R_1$ and $R_2$ individually or in combination mean a protecting group for a hydroxyl group, $R_3$ denotes a protected amino group and $R_4$ represents:

group 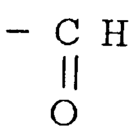 or group 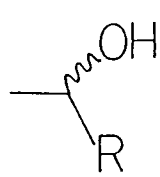

wherein R is a lower alkyl group or a lower alkynyl group.

European Patent Office

# EUROPEAN SEARCH REPORT

Application Number

EP 91115578.6

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 109, no. 25, December 19, 1988 Columbus, Ohio, USA V. E. MARGUEZ et al. "Total synthesis of (-)-neplanocin A." pages 908,909, abstract-no. 231 460k & J. Org. Chem. 1988, 53(24), 5709-14 -- | 1-6 | C 07 D 473/34 |
| A | CHEMICAL ABSTRACTS, vol. 105, no. 7, August 18, 1986 Columbus, Ohio, USA A. HOSHI et al. "Antitumor activity of derivatives of neplanocin A in vivo and in vitro." pages 15,16, abstract-no. 54 107v & Pharmacobio-Dyn. 1986, 9(2), 202-6 ---- | 1-6 | |

TECHNICAL FIELDS SEARCHED (Int. Cl.5)

C 07 D 473/00

The present search report has been drawn up for all claims

| Place of search | Date of completion of the search | Examiner |
|---|---|---|
| VIENNA | 13-12-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)